# EUROPEAN PATENT APPLICATION

(11) **EP 4 691 480 A1**
(43) Date of publication of application: **11.02.2026**
(21) Application number: 23929031.5
(22) Date of filing: 28.11.2023
(51) Int. Cl.: A61K 38/02, A61K 38/04, A61P 35/00, C07K 11/00, A61K 38/00

(54) **CONNEXIN-46 PEPTIDE MODULATOR**

(30) Priority: 28.03.2023 US 202318191023
(71) Applicant: Universidad del Desarrollo, Santiago (CL)
(72) Inventor: RETAMAL LUCERO, Mauricio Antonio, Las Condes, Santiago (CL)
(74) Representative: Balder IP Law, S.L.
(86) International application number: PCT/CL2023/050117
(87) International publication number: WO 2024/197425

(57) **Abstract**

The present invention provides mimetic peptides that modulate connexin-46 (Cx46), pharmaceutical compositions comprising said mimetic peptides, and methods for treating cancer comprising administering to a subject in need a therapeutically effective amount of the Cx46 mimetic peptide or the pharmaceutical composition containing a Cx46 mimetic peptide.

## Description

### TECHNICAL FIELD

The present invention relates to connexin modulators, specifically peptides and/or peptidomimetics that modulate connexin-46, which are useful as a cancer therapeutic agent.

### BACKGROUND

Connexins (Cxs) are transmembrane proteins whose primary function is to allow intercellular communication by forming hemichannels and gap junctions. Under normal conditions, hemichannels allow the controlled release of signaling molecules to the extracellular milieu. However, hemichannels can induce and/or exacerbate symptoms under pathological conditions.

There are 21 human genes coding different connexins (Cxs) isoforms, which are expressed in practically all cell types. Among those, Cx46 stands out for its restricted expression pattern since, in humans, it has only been described in the eye lens. It has been reported that mutations in Cx46 and Cx50 are associated with lens malfunction and cataract formation (Retamal, M. A., et al. (2015). Diseases associated with leaky hemichannels. Frontiers in cellular neuroscience, 9, 267; Retamal, M. A. & Altenberg, G. A. (2022). Role and Posttranslational Regulation of Cx46 Hemichannels and Gap Junction Channels in the Eye Lens. Frontiers in Physiology, 13, 864948; Retamal, M. A., et al. (2019). Cx46 hemichannel modulation by nitric oxide: Role of the fourth transmembrane helix cysteine and its possible involvement in cataract formation. Nitric Oxide, 86, 54-62). Recently, the presence of Cx46 has also been reported in breast carcinoma, melanoma, and glioblastoma multiforme (GBM) (Hitomi, M., et al. (2015). Differential Connexin Function Enhances Self-Renewal in Glioblastoma. Cell Reports, 11(7), 1031-1042.; Acuña, R. A., et al. (2020). Connexin-46 Contained in Extracellular Vesicles Enhance Malignancy Features in Breast Cancer Cells. Biomolecules, 10(5), 676.; Acuña, R. A., et al. (2021). Connexin46 Expression Enhances Cancer Stem Cell and Epithelial-to-Mesenchymal Transition Characteristics of Human Breast Cancer MCF-7 Cells. International journal of molecular sciences, 22(22), 12604; Orellana, V. P., et al. (2020). Connexins in melanoma: Potential role of Cx46 in its aggressiveness. Pigment Cell Melanoma Research, 34, 853-868).

Therefore, Cxs are possible therapeutic targets for different types of diseases. In the last decade, small molecules, peptides, and antibodies have been developed to modulate connexins hemichannels (Retamal, M. A., et al. (2021). Over-activated hemichannels: A possible therapeutic target for human diseases. BBA - Molecular Basis of Disease, 1867, 166232). For example, document US 2018/0008664 A1 discloses compounds and compositions comprising a gap junction or hemichannel blocker or inhibitor for treating or preventing vascular and other diseases. Said blockers or inhibitors are peptides and mimetic peptides that bind to gap junction and hemichannel proteins. Document WO 2006/134494 A2 discloses anti-connexin compounds, compositions, formulations, and methods for modulation of gap junctions and hemichannels that are useful, for example, in the prevention and treatment of various diseases such as cardiovascular, neurological, and vascular diseases. Document CN108066744A discloses the use of Cx43 mimetic peptide Gap19 for treating cerebral hemorrhage. Document US 2017/0128475 A1 discloses a composition and methods for treating the inflammatory response in a tissue or organ, such as the liver, by contacting the tissue with a tissue-specific gap junction inhibitor, wherein the inhibitor could be a Cx32 mimetic peptide. However, there are no reports about mimetic peptides directed against Cx46 for its use in the treatment of cancer.

### SUMMARY OF THE INVENTION

The present invention provides mimetic peptides that modulate connexin-46 (Cx46), pharmaceutical compositions comprising said mimetic peptides, and methods for treating cancer comprising administering to a subject in need a therapeutically effective amount of the Cx46 mimetic peptide or the pharmaceutical composition containing a Cx46 mimetic peptide.

In one embodiment, the mimetic peptide has an amino acid sequence having 75% or more, 80% or more, 85% or more, 95% or more, 99% or more, amino acid sequence similarity to any of the amino acid sequences set forth in SEQ ID NO: 2 to SEQ ID NO: 7. In a preferred embodiment, the mimetic peptide has any of the amino acid sequences set forth in SEQ ID NO: 2, SEQ ID NO: 3, SEQ ID NO: 4, SEQ ID NO: 5, SEQ ID NO: 6, or SEQ ID NO: 7.

In one embodiment, the pharmaceutical composition comprises a mimetic peptide that has an amino acid sequence having at least 75%, at least 80%, at least 85%, at least 95%, at least 99%, amino acid sequence similarity to any of the amino acid sequences set forth in SEQ ID NO: 2 to SEQ ID NO: 7. In a preferred embodiment, the pharmaceutical composition comprises a mimetic peptide selected from the group consisting of the amino acid sequences set forth in SEQ ID NO: 2, SEQ ID NO: 3, SEQ ID NO: 4, SEQ ID NO: 5, SEQ ID NO: 6, and SEQ ID NO: 7. In another embodiment, the pharmaceutical composition comprises one or more of the above-defined mimetic peptides and a pharmaceutically acceptable excipient.

In one embodiment, the present invention also provides a mimetic peptide comprising an amino acid sequence having 75% or more, 80% or more, 85% or more, 95% or more, 99% or more, 100% amino acid sequence similarity to any of the amino acid sequences set forth in SEQ ID NO: 2 to SEQ ID NO: 7 for use in the treatment of cancer.

### BRIEF DESCRIPTION OF THE FIGURES

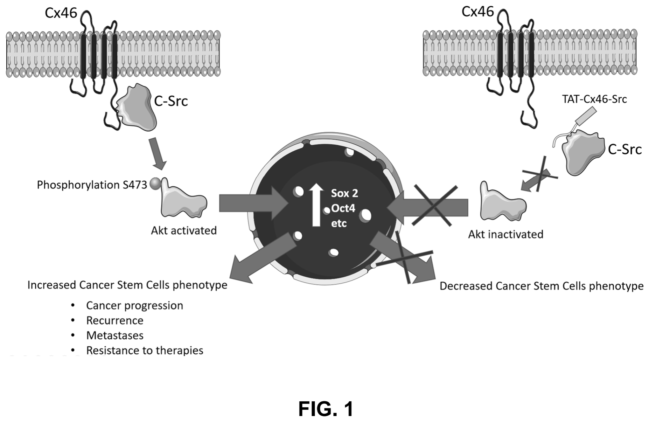

FIG. 1 shows a hypothetical scheme of the mode of action of TAT-Cx46-Src. Cx46 through its C-terminal has the capacity to interact with c-Src which in turn can modulate the activity of Akt, which is a key protein setting up the Cancer stem cells characteristic of cancer cells (stemness). Thus, Akt activated can promote an increase of the transcription of genes associated to cancer progression, recurrence, resistance to therapies, and metastases. Our peptide (TAT-Cx46-Src) is able to block the interaction between Cx46 and c-Src, which in turn will 1) decrease Akt activation and 2) decrease the stemness of cancer cells.

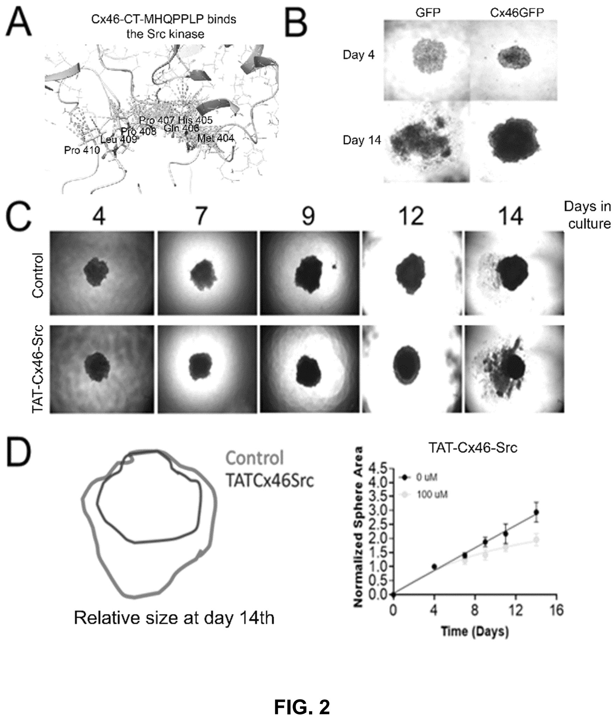

FIG. 2 (A) Shows how trough molecular docking studies, it was determined that a sequence of Cx46 C-terminus has the potential to interact with Cx46 C-terminal. (B) Tumor spheres growing is a technique used to determine the cancer stem cells capacity. Hela cells transfected with GFP for a monolayer of cells when they a seeded in a non-adherent surface, and moreover they died after 14 days in culture. However, Hela cells transfected with Cx46GFP do form tumor spheres which survive at last for 14 days. (C) Addition of 100 µM TAT-Cx46-Src to the extracellular media every 3 days, reduced tumor sphere size. (D) graphical comparison between the size of tumor spheres at day 14 under control conditions (outer line) and treated with the peptide (inner line). Plot show the quantification of the tumor sphere size between time 0 and day 14, both under control condition (upper line) and with the peptide (lower line).

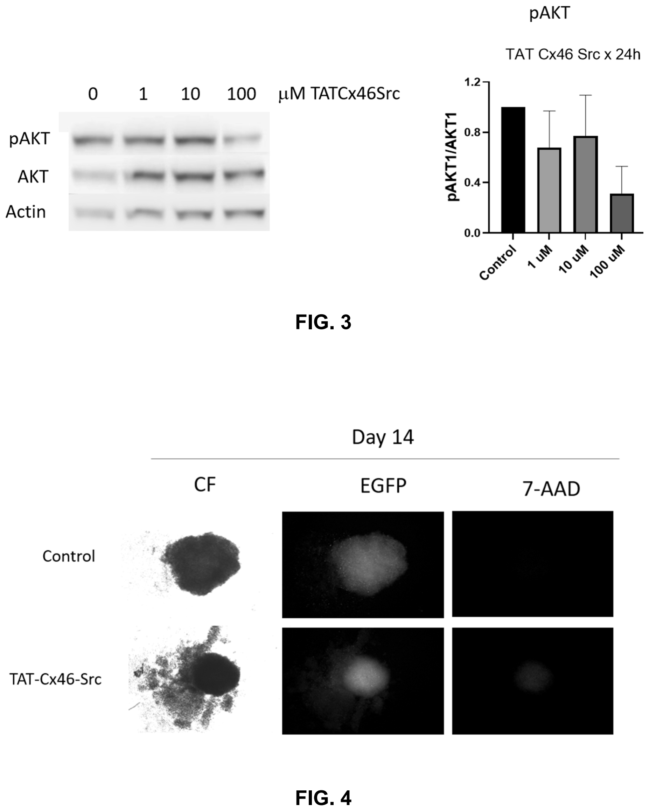

FIG. 3 Shows that TAT-Cx46-Src decreased Akt phosphorylation in S473. (A) Representative Western blot showing, the levels of phosphorylation at S473 (pAKT), total AKT (AKT) and loading control (actin). Different concentrations of TAT-Cx46-Src were tested, ranging between 0 - 100 µM. (B) Plot showing the relative levels of pAKT with respect to total AKT at different concentrations of TAT-Cx46-Src. (n=3).
FIG. 4 Shows that TAT-Cx46-Src increase cell death of Hela cells Cx46GFP. Tumor spheres formed by HeLa cells transfected with Cx46GFP were grown for 14 days. 100 µM TAT-Cx46-Src was added every 3 days (every time that culture media was replaced). At day 14th, 10 mM 7-AAD was added to the extracellular media. Only cells treated with TAT-Cx46-Src shown uptake of this Dye, suggesting that these cells present a compromised plasma membrane, that allow the entry of this large and non-permeable molecule to their plasma membrane (n=3).

### DETAILED DESCRIPTION OF THE INVENTION

The present invention provides mimetic peptides that modulate connexin-46 (Cx46). Surprisingly, the mimetic peptides disclosed herein are useful for treating the different types of cancer, such as breast carcinoma, melanoma, glioblastoma multiforme (GBM), among others, and/or preventing the progression, recurrence, metastasis, and resistance to antitumor therapies. The mimetic peptides of the present invention could also be used as co-treatment with the current anti-cancer therapies to improve their antitumor effects.

Unless defined otherwise, all technical and scientific terms used herein have the same meaning as commonly understood by those of ordinary skill in the art to which the invention belongs. Various terms are defined below to facilitate the understanding of the present invention but are not intended to be limiting.

The term "amino acid" refers to natural amino acids, unnatural amino acids, and amino acid analogs. The term "amino acid analog" refers to a natural or unnatural amino acid where one or more of the C-terminal carboxy group, the N-terminal amino group and side-chain functional group has been chemically blocked, reversibly or irreversibly, or otherwise modified to another functional group.

As used herein, the term "peptide" refers a short polymer of amino acids linked together by peptide bonds. In contrast to other amino acid polymers (e.g., proteins, polypeptides, etc.), peptides are of about 50 amino acids or less in length. A peptide can comprise natural amino acids, non-natural amino acids, amino acid analogs, and/or modified amino acids. A peptide can be a subsequence of naturally occurring protein or a non-natural (synthetic) sequence.

The terms "mimetic peptide", "peptidomimetic" and "peptide modulator" are used interchangeably herein and refer to a peptide-like molecule that emulates a sequence derived from a protein or peptide and their function. This definition includes chimeric peptides also called fusion proteins, consisting of the union of two or more sequences; peptides with chemical modifications to their amino acids; and peptides with non-natural amino acids incorporated.

The terms "connexin-46", "connexin 46" and "Cx46" are used interchangeably herein and are referred to a gap junction protein, specifically to the gap junction alpha-3 protein (GJA3), preferably from *Homo sapiens* as disclosed in SEQ ID NO: 1 or annotated in the NCBI protein database under the accession number NP_068773.

The term "sequence similarity" refers to the degree to which two polymer sequences (e.g., peptide, polypeptide, nucleic acid, etc.) differ only by conservative and/or semi-conservative amino acid substitutions. The term "conservative substitution", as used herein, denotes that another biologically similar residue replaces one or more amino acids. Examples include the substitution of amino acid residues with similar characteristics, e.g., small amino acids, acidic amino acids, polar amino acids, basic amino acids, hydrophobic amino acids, and aromatic amino acids. The "percent sequence similarity" is calculated by: (1) comparing two optimally aligned sequences over a window of comparison (e.g., the length of the longer sequence, the length of the shorter sequence, a specified window, etc.), (2) determining the number of positions containing identical (or similar) monomers (e.g., same amino acids occur in both sequences, similar amino acid occurs in both sequences) to yield the number of matched positions, (3) dividing the number of matched positions by the total number of positions in the comparison window (e.g., the length of the longer sequence, the length of the shorter sequence, a specified window), and (4) multiplying the result by 100 to yield the percent sequence identity or percent sequence similarity.

The term "subject" broadly refers to an animal, including, but not limited to, a primate (e.g., human), cow, sheep, goat, horse, dog, cat, rabbit, rat, or mouse. The terms "subject" and "patient" are used interchangeably herein, for example, to a mammalian subject, such as a human subject.

The terms "treat", "treating", and "treatment" can be meant to include alleviating or abrogating a disorder, disease, or condition; or one or more of the symptoms associated with the disorder, disease, or condition; or alleviating or eradicating the cause(s) of the disorder, disease, or condition itself. Desirable effects of treatment can include, but are not limited to, preventing occurrence or recurrence of disease, alleviation of symptoms, diminishing any direct or indirect pathological consequences of the disease, preventing metastasis, decreasing the rate of disease progression, amelioration or palliation of the disease state and remission or improved prognosis.

The term "therapeutically effective amount", "therapeutically effective dose", "effective amount" or "effective dose" refers to the amount of a compound that, when administered, can be sufficient to prevent the development of, or alleviate to some extent, one or more of the symptoms of the disorder, disease, or condition being treated. The term "therapeutically effective amount" can also refer to the amount of a compound that is sufficient to elicit the biological or medical response of a cell, tissue, system, animal, or human that is being sought by a researcher, veterinarian, medical doctor, or clinician.

The term "pharmaceutically acceptable excipient" refers to a pharmaceutically-acceptable material, composition, or vehicle, such as a liquid or solid filler, diluent, excipient, solvent, or encapsulating material. A component can be "pharmaceutically acceptable" in the sense of being compatible with the other ingredients of a pharmaceutical formulation. It can also be suitable for use in contact with the tissue or organ of humans and animals without excessive toxicity, irritation, allergic response, immunogenicity, or other problems or complications, commensurate with a reasonable benefit/risk ratio.

A "cancer therapeutic agent" or "anti-cancer agent", as used herein, can refer to an agent or therapy that is capable of negatively affecting cancer in a subject, for example, by killing cancer cells, inducing apoptosis in cancer cells, reducing the growth rate of cancer cells, reducing the incidence or number of metastases, reducing tumor size, inhibiting tumor growth, reducing the blood supply to a tumor or cancer cells, promoting an immune response against cancer cells or a tumor, preventing or inhibiting the progression of cancer, or increasing the lifespan of a subject with cancer.

In one embodiment, the Cx46 mimetic peptide of the present invention has an amino acid sequence having 75% or more, 80% or more, 85% or more, 95% or more, 99% or more, 100% amino acid sequence similarity to any of the amino acid sequences set forth in SEQ ID NO: 2 to SEQ ID NO: 7. In a preferred embodiment, the mimetic peptide having between 75 - 100% amino acid sequence similarity to any of the amino acid sequences set forth in SEQ ID NO: 2, SEQ ID NO: 3 and SEQ ID NO: 4 may include a TAT peptide sequence (GRKKRRQRRR set forth in SEQ ID NO: 8) that facilitates the internalization of the mimetic peptide into the cell. The mimetic peptides, including said TAT peptide sequence, are shown in SEQ ID NO: 5, SEQ ID NO: 6 and SEQ ID NO: 7.

In one embodiment, the pharmaceutical composition comprises a Cx46 mimetic peptide that has an amino acid sequence having 75% or more, 80% or more, 85% or more, 95% or more, 99% or more, amino acid sequence similarity to any of the amino acid sequences set forth in SEQ ID NO: 2 to SEQ ID NO: 7. In a preferred embodiment, the pharmaceutical composition comprises a mimetic peptide selected from the group consisting of the amino acid sequences set forth in SEQ ID NO: 2, SEQ ID NO: 3, SEQ ID NO: 4, SEQ ID NO: 5, SEQ ID NO: 6, and SEQ ID NO: 7. In another embodiment, the pharmaceutical composition comprises one or more of the above-defined mimetic peptides and a pharmaceutically acceptable excipient.

In some embodiments, the pharmaceutically acceptable excipient could be a carrier, diluent, or buffer, and it is suitable for use in a subject. Such excipients include any pharmaceutical agent that can be administered without undue toxicity. The pharmaceutically acceptable excipients may include salts, for example, mineral acid salts such as hydrochlorides, hydrobromides, phosphates, sulfates, and the like; and the salts of organic acids such as acetates, propionates, malonates, benzoates, and the like. Additionally, auxiliary substances, such as wetting or emulsifying agents, pH buffering substances, and the like, may be present in such vehicles. A wide variety of pharmaceutically acceptable excipients are known in the art and need not be discussed in detail herein. Pharmaceutically acceptable excipients have been amply described in a variety of publications, including, for example, Allen L, Popovich N, Ansel H. (2011). Ansel's Pharmaceutical Dosage Forms and Drug Delivery Systems. 9th edition. Lippincott Williams & Wilkins, y Rowe R, Sheskey P, Quinn M. (2009). Handbook of Pharmaceutical Excipients. 6th edition. Pharmaceutical Press, which are incorporated herein by reference.

The pharmaceutical composition of the present invention can be applied by all known routes of administration, for example, by injection and/or parenteral route (for example, intravenous, intraarterial, intramuscular, intraperitoneal, intradermal, subcutaneous, by direct injection into various organs, among others, without being limited to other alternatives), by inhalation, through continuous release pumps, suppositories, orally, among others, without being limited to those mentioned here. Said administration can be in a single, multiple doses, or continuous administration.

The pharmaceutical composition of the present invention may be in liquid, solid, semi-solid form, or a mixture thereof, including tablets, wafers, dragees, tablets, capsules, pills, lozenges, powder, aqueous or oily suspensions, solutions, creams, ointments, coated formulations, sustained release formulations, aerosols, micro and/or nanoparticles, among others, without being limited to those mentioned here.

In one embodiment, the present invention also provides a Cx46 mimetic peptide comprising an amino acid sequence having 75% or more, 80% or more, 85% or more, 95% or more, 99% or more, 100% amino acid sequence similarity to any of the amino acid sequences set forth in SEQ ID NO: 2 to SEQ ID NO: 7 for use in the treatment of cancer and/or preventing the progression, recurrence, metastasis, and resistance to antitumor therapies. In a preferred embodiment, the mimetic peptide is useful in the treatment of breast carcinoma, melanoma, and/or glioblastoma multiforme (GBM), without limitation to these mentioned.

In another embodiment, the present invention includes a method for treating cancer and/or preventing the progression, recurrence, metastasis, and resistance to antitumor therapies comprising administering to a subject in need a therapeutically effective amount of a Cx46 mimetic peptide comprising an amino acid sequence having 75% or more, 80% or more, 85% or more, 95% or more, 99% or more, 100% amino acid sequence similarity to any of the amino acid sequences set forth in SEQ ID NO: 2 to SEQ ID NO: 7, or a pharmaceutical composition including said Cx46 mimetic peptide. In an embodiment, the method of the present invention is for treating breast carcinoma, melanoma, glioblastoma multiforme (GBM), without limiting to these mentioned. The therapeutically effective amount of the Cx46 mimetic peptide or the pharmaceutical composition comprising said Cx46 mimetic peptide can be administered in one or more administrations.

The present invention is further described below with reference to specific examples. It should be understood that these examples are intended only to illustrate the invention and are not intended to limit the scope of the invention.

### EXAMPLES

### Example 1. Cx46 mimetic peptide designs

Mimetic peptides of the Cx46 domains that would interact with c-Src, Akt or PI3k were designed, synthesized and evaluated for their ability to inhibit the formation of tumor spheres *in vitro* in HeLa cells overexpressing or not Cx46. Briefly, the experiments were done with the following protocols.

Cell line cultures: The HeLa cell line was grown in Dulbecco's Modified Eagle Medium (DMEM) supplemented with 10% fetal bovine serum (FBS) and 1% penicillin 10,000 U/mL, streptomycin sulfate 100 mg/mL (P/S) at 37 °C, 5% CO² and 95% humidity. Every 2 weeks the presence of mycoplasma was analyzed, by means of staining with DAPI. Infected cells were treated with BioMyc-1 (100x) for two weeks.

Cell Transfection: Cells were transfected using Lipofectamine 2000 (Invitrogen). For this HeLa cell cultures were grown to reach 60% confluence. Cells are washed with serum-free DMEM twice and finally exposed to OptiMem^{™} (Invitrogen) plus 1 µg cDNA. The cDNA for GFP-linked human Cx46 was purchased from Promega. The plasmid also has a resistance to G418, which is an antibiotic that serves for the selection of cells that stably express Cx46-GFP. Cx46 expression was monitored by Western blot and immunofluorescence analysis.

Western blots: Cells were washed three times with phosphate buffered saline (PBS) pH: 7.4 and then lysed with RIPA buffer (150 mM NaCl, 20 mM Tris pH: 7.4; 0.5% Sodium Desoxycholate, 0.1% Sodium Dodecyl Sulfate (SDS), 1% Nonidet P -40 and a protease inhibitor cocktail tablet). Proteins were quantified using the Qubit^{™} protein Assay Kit in a Qubit^{™} Fluorometer following the manufacturer's instructions (ThermoFisher). Subsequently, Laemli buffer (4x) (62.5mM Tris pH 6.8; 25% glycerol, 2% SDS, 0.01% bromophenol blue and 5% β-mercaptoethanol) was added to the cell lysate and was heated at 95°C for 3 min, in a thermoblock. About 50 µg of proteins were loaded into each well of a 10% polyacrylamide gel under denaturing conditions (SDS-PAGE). Then the proteins were transferred to a polyvinylidene difluoride (PVDF) membrane by wet transfer for 2 h at 100 V in Tris-Glycine 20% methanol buffer. The membranes were blocked with 5% skimmed milk in Buffer Tris and 0.05% Tween 20 (T-TBS) for 1 h at room temperature under shaking. After blocking, the membranes were incubated overnight at 4°C with the primary antibodies. Subsequently, the membranes were washed 3 times with 0.05% T-TBS for 10 min and incubated with the goat anti-mouse or goat anti-rabbit secondary antibody as appropriate, both conjugated with peroxidase (HRP) for 1 h under shaking at room temperature. After three washes with T-TBS, the membranes were revealed by chemiluminescence using a C-Digit Blot Scanner. The primary antibodies used were anti-Cx46 (C3; Santa Cruz Biotechnology), anti-Akt (G.145.7; Invitrogen); anti-pAkt (S473; 14-6, Invitrogen); anti-Src (MAB3389, R&D Systems); anti-pSrc (Y416; MAB2685, R&D Systems). As a loading control, a primary anti-β-actin antibody (SC-47778, Santa Cruz Biotechnology) was used.

Peptides were designed as follow; The crystallized structures of the c-Src, was taken from PDB DATABANK (https://www.rcsb.org), corresponding to input 2H8H, with a 2.2å input resolution. On the other hand, the amino acid sequence of human Cx46 was obtained from Uniprot (https://www.uniprot.org), corresponding to the entry Q9y6h8 and the structure of the C-Terminal of Cx46 (from 141 to 435 aa) was modeled using the I-Tasser server (https://zhangglab.ccmb.med.umich.edu/tasser/). Final structures were obtained from the analyzes with Maestro Schrodinger software and the optimization with PROPKA software at pH 7.0. Then, the possible sites of interaction were determined using SiteMap and Protein-protein Docking PiPER software. Thus, was determined that the sequence MHQPPLP of the Cx46 C-terminal has possible interactions with c-Src. With is information, we designed a peptide containing this sequence in order to block the possible interaction between Cx46 C-terminal and c-Src.

Interestingly, only the Cx46 CT segment mimetic peptide (Cx46404-410) with high potential for interaction with c-Src (FIG 2A; Annex 1) showed strong inhibition of the cancer stem cells (CSC) functional phenotype in HeLa cells (FIG 2C and D; Annex 1). This mimetic peptide of the Cx46404-410 segment was designed to be able to cross the plasma membrane and compete with the interaction between c-Src and Cx46. Cellular permeability was increased by fusion with TAT, a cell-penetrating peptide derived from the transactivator of HIV53 transcription. The mimetic peptide with the capability of crossing the plasma membrane was called TAT-Cx46-Src (FIG 4; Annex 1) and its amino acid sequence is shown in SEQ ID NO: 7.

Interestingly, it was observed that 24-h treatment of HeLa Cx46-GFP cells with the peptide TAT-Cx46-Src (100 µM) strongly decreased Akt phosphorylation levels in S473 (FIG 3A and B; Annex 1). Tumor sphere formation studies revealed that HeLa cells transfected with GFP (transfection control) only form a monolayer of cells at the bottom of a non-adherent agar plate on day 4 post-seeding and these do not survive beyond 14 days, that is, they do not have the capacity to form tumor spheres. However, HeLa cells transfected with Cx46-GFP form large tumor spheres that remain alive for at least 14 days (FIG 2B; Annex 1). These results indicate, once again, that Cx46 expression promotes an increase in CSC characteristics, since only this class of cells is capable of forming tumor spheres on non-adherent surfaces. Importantly, it was observed that 10 days of treatment with TAT-Cx46-Src (100 µM) decreased the size of tumor spheres formed by HeLa Cx46-GFP cells by about 43% (FIG 2D; Annex 1).

Additionally, cell viability studies show that this treatment promotes the death of cells in the center of tumor spheres (FIG 4; Annex 1). All these data allow to hypothesize that the TAT-Cx46-Src peptide would interfere with the interaction between Cx46 and c-Src, consequently inhibiting the activation of c-Src mediated by Cx46 and, in this way, would suppress both Akt activation and c-Src activation, and the consequent increase in CSC characteristics in tumor cell cultures (FIG. 1).

## Claims

1. A mimetic peptide that modulates connexin-46, comprising an amino acid sequence having at least 75% amino acid sequence similarity to any of the amino acid sequences set forth in SEQ ID NO: 2 to SEQ ID NO: 7.

2. The mimetic peptide according to claim 1, wherein the peptide comprises any of the amino acid sequences set forth in SEQ ID NO: 2 to SEQ ID NO: 7.

3. The mimetic peptide according to claim 1, to be used in the treatment of cancer.

4. The mimetic peptide according to claim 3, wherein the cancer is breast carcinoma, melanoma, or glioblastoma multiforme (GBM).

5. A pharmaceutical composition, comprising at least one mimetic peptide that modulates connexin-46 and a pharmaceutically acceptable excipient, wherein said mimetic peptide comprises an amino acid sequence having at least 75% amino acid sequence similarity to any of the amino acid sequences set forth in SEQ ID NO: 2 to SEQ ID NO: 7.

6. The pharmaceutical composition according to claim 5, wherein the mimetic peptide comprises any of the amino acid sequences set forth in SEQ ID NO: 2 to SEQ ID NO: 7.

7. A method for the treating cancer comprising administering to a subject in need a therapeutically effective amount of a mimetic peptide that modulates connexin-46 or a pharmaceutical composition comprising said mimetic peptide, wherein the mimetic peptide comprises an amino acid sequence having at least 75% amino acid sequence similarity to any of the amino acid sequences set forth in SEQ ID NO: 2 to SEQ ID NO: 7.
